# EUROPEAN PATENT SPECIFICATION

(11) **EP 1 957 119 B1**
(45) Date of publication and mention of the grant of the patent: **13.06.2012**
(21) Application number: 06835644.3
(22) Date of filing: 01.12.2006
(51) Int. Cl.: A61L 9/03, A61L 9/12, F24F 3/16

(54) **FRAGRANCE DELIVERY SYSTEM**
DUFTABGABESYSTEM
SYSTEME DE DIFFUSION DE PARFUM

(30) Priority: 01.12.2005 NL 1030571; 01.12.2005 WO PCT/NL2005/000823; 01.12.2005 US 741062 P
(43) Date of publication of application: 20.08.2008
(73) Proprietor: The Procter & Gamble Company, Cincinnati, OH 45202 (US)
(72) Inventor: BRONCANO ATENCIA, Toni, Barcelona (ES); SORRIBES, Silvia, E-08221 Barcelona (ES); GÜELL, Francisco, E-08190 Barcelona (ES)
(74) Representative: Morelle, Evelyne Charlotte Isabelle
(86) International application number: PCT/NL2006/000602
(87) International publication number: WO 2007/064197

(56) References cited:
- EP-A- 1 543 844
- WO-A-2004/043502
- GB-A- 2 347 860
- US-A1- 2002 159 916
- US-A1- 2005 094 988

## Description

The present invention relates to a fragrance delivery system, and a kit of parts comprising said device and at least two fragrance reservoirs filled with a fragrance substance.

Fragrance dispersion systems are as such known in the art. Many of such systems are directed to the prevention of the habituation of a fragrance composition. The problem encountered in terms of fragrance habituation is that a person in a particular space will become accustomed to a particular fragrance after a period of time.

Whereas many known systems apply just one single fragrance, recently fragrance delivery systems have been developed that contain two fragrances which are delivered in an alternating manner, one after the other. One such fragrance delivery system is known from EP 1 543 844 A. Such delivery systems have, however, various drawbacks. They deal not in an effective manner with the problem of fragrance habituation, since the first fragrance is automatically followed by the second fragrance at a same intensity.

It is further noted that documents GB 2 347 860 A, WO 2004/043502 A, US 2005/094988 A1, and US 2002/159916 A1 relate to known fragrance delivery systems that contain two or more fragrances.

GB 2 347 860 A discloses a fragrance dispenser having a microprocessor for controlling heaters, the control unit comprising a programmable chip and a dual timer for switching between a first state and a second state. A main fragrance can be dispensed continuously, while subsidiary fragrances can be dispensed at controlled four hour periods.

Document WO 2004/043502 A discloses a fragrance delivery system with a controlling unit for controlling release of fragrances. The controlling unit comprises a user interface for selecting fragrances, a memory card and intensity level control knobs for switching the release mechanism. This fragrance delivery system allows emission in spaced-apart bursts and alternating use of similarly themed fragrances over consecutive set periods of time to avoid fragrance habituation.

Document US 2005/094988 A1 discloses a multi-fragrance scent dispenser with a control circuit for selectively controlling activation of different heaters associated with different scent materials. Scent habituation is counteracted by terminating power to one heater and activating a next heater.

US 2002/159916 A1 copes with the problem of scent habituation by providing a system that comprises a controller arranged for supplying heat continuously to a first heater and periodically to a second heater associated with a different fragrance than the first heater.

Object of the present invention is to provide a fragrance delivery systems which deals with the problem of fragrance habituation in an effective manner, whereas at the same time it provides flexibility in the sense that the fragrance intensity may be adjusted to meet more appropriately the size of the space to which the fragrance is to be delivered.

Surprisingly, it has been found that these objects may be realised when use is made of a fragrance delivery system which allows each of the fragrances to be released periodically.

Accordingly, the present invention relates to a fragrance delivery system comprising:
a control unit for controlling a release mechanism to release at least one selected fragrance, said control unit comprising a selection unit for selecting said selected fragrance from at least two fragrances and controlling said release mechanism to release said selected fragrance, and a switching element for switching said release mechanism between an first state and an second state or vice versa, in which first state said release mechanism releases said selected fragrance at a higher rate than in said second state, characterised in that said switching element is arranged to switch said release mechanism between said first state and said second state during a first period of time, and to switch said release mechanism between said first state and said second state during a second period of time, in which second period of time the ratio of the duration of the first state relative to the duration of the second state is lower than in the first period of time, wherein said first period and said second period follow in an alternating manner, and wherein the second period is sufficiently long to reduce the detection level of a human being, such as to the level before habitation. The fragrance delivery system according to the present invention has the advantage that the fragrance may be delivered at a desired intensity, taking the size of the space to which the fragrance needs to be delivered into account.

Suitably, said selection unit is arranged to periodically select, in response to a selection signal, instead of a presently selected fragrance another selected fragrance, which delivery system further comprises a timer for measuring a period of time during which said presently selected fragrance has been released, and a comparator for comparing said measured period with a predetermined value representing a desired period and outputting said selection signal to the selection unit.

In this way a sequence of selected fragrances can be released. In accordance with the present invention at least one fragrance can be selected from at least two fragrances. Preferably, one fragrance is selected from at least two fragrances. More preferably, one fragrance is selected from three fragrances.

Suitably, the present delivery system a further comprises a timer for measuring a period of time of said first state and/or second state, a memory element in which at least one desired value of a period of the first and/or second state can be stored, and a comparator for comparing the measured period with said desired value and outputting a switching signal to said switching element, and wherein said switching element is arranged to change said state in response to said switching signal.

Suitable values for the combined first and second period are found to be in the range of 15 to 60 minutes. The first period has suitably a longer duration than said second period. For example, the first period may have a duration of more than 15 minutes, such as between 20 and 60 minutes, and/or in less than or equal to 60 minutes. The second period may for example have a duration of less than 30 minutes, such as between 10 and 25 minutes.

Suitably, the selected fragrance can be released during both the first period of time and the second period of time in a continuous manner. When a lower overall fragrance intensity is required, each of the selected fragrances can suitably be released in a series of first states and second states, in the form of pulses. Hence, the first state will be switched to the second state, after which the second state can be switched to the first state. The period of time in which such a series of the first states and the second states can be switched from one to the other is suitably in the range of from 15 to 60 minutes, preferably in the range of from 20 to 40 minutes, more preferably in the range of from 25 to 35 minutes. During such a period of time the duration of the first states is preferably in the range of from 100 to 200 seconds, and the duration of the second states is preferably in the range of from 20 to 100 seconds.

Suitably, the release mechanism can be disconnected or switched off after each first state to ensure that less fragrance will be released during each second state.

The last first state in such a series of first and second states can be followed up with a single second state of which the duration is preferably in the range of from 5 to 30 minutes, more preferably in the range of from 10 to 20 minutes. During such a single second state the release of the selected fragrance will be less than the release during the preceding first state. Suitably, said release mechanism can be disconnected or switched off to ensure that less or no fragrance will be released during such a single second state. Such a single second state can suitably be followed up with a single first state of another selected fragrance or a series of first states and second states of another selected fragrance. Such a single first state or the last first state in a series of first and second states of the other fragrance can in turn be followed by another single second state, after which a single first state or a series of first and second states can be applied of yet another selected fragrance.

The duration of the first and second states can suitably be adjusted so as to bring about an overall fragrance intensity which is appropriate for the space into which the fragrances(s) is (are) released. The overall fragrance intensity will become higher when the duration of the first states becomes longer, whereas it will decrease when the duration of the second state(s) become(s) longer.

Suitably, the duration of the respective first and second states during the period that a particular fragrance is released can be varied. Suitably, at least two sets of desired values of the durations of the first and second state(s) are provide, preferably three sets of desired values of the durations of the first and second state(s). In one such a set the duration of the first states in a series of first and second states may be 200 seconds and the duration of the second states may be 40 seconds, whereas in another set the duration of the first states may be 150 seconds and the duration of the second states may be 100 seconds. It will be understood that the overall fragrance intensity as experienced by an individual will be higher with the former set than with the latter set. Thus, the present invention provides flexibility to ensure that the overall fragrance intensity as experienced by an individual can be adjusted to meet appropriately the size of the space to which the fragrance(s) is (are) released.

Suitably, the fragrance delivery system has three modes of operation, namely two modes of operation which provide lower overall fragrance intensities in which each mode use is made of a different set of desired values of the duration of the series of first and second states, whereas in a third mode of operation in the first period and second period of time the fragrance is released continuously, without pulses, providing a maximum overall fragrance intensity.

Suitably, said first period has a longer duration than said second period.

During the second period of time, said release mechanism is suitably in the second state only.

Suitably, in the second state said release mechanism releases said selected fragrance at a rate below a threshold at which an individual notices said fragrance, such as a rate of substantially zero.

Preferably, said selection unit is arranged to periodically select a selected fragrance.

Preferably, said selection unit is arranged to select a selected fragrance when a second period expires.

Suitably, said release mechanism includes an electro-thermal transducer, said transducer being electrically connectable to a electrical power source and which transducer can be coupled thermally to a fragrance substance, for converting electrical energy from said power source to thermal energy applied to said selected fragrance.

The present delivery system suitably further comprising a holder for holding at least two fragrance containers.

Suitably, a release mechanism engageable on said fragrance containers, for releasing a selected one of at least two fragrances from said fragrance container.
The timer is capable of measuring a first period of time in the range of from 100 to 200 seconds, and a second period of time in the range of from 20 to 100 seconds. Suitably, the fragrance delivery system further comprising a release mechanism for releasing a selected one of at least two fragrances from said fragrance container. Preferably, the release mechanism comprises a heater which allows the fragrance to be released from the container at a particular temperature.

Suitably, the heater will be heated to the active state enabling the fragrance to vaporize, whereas the heater will be switched off during the inactive state.

The heater can be of any form, for instance, in the form of a ring or a coil around the wick means of the container containing the fragrance substance.

The selection unit is arranged in such a way that the at least two fragrances are selected in an alternating manner.

Such release mechanism can either be part of the holder for holding the at least two fragrance containers or at may be part of each of the fragrance containers. Accordingly, the at least two fragrance containers can suitably each be provided with a release mechanism to release the fragrance contained in the fragrance container.

Preferably, the fragrance delivery system according to the present invention is capable of releasing at least three different fragrances in an alternating manner.

The fragrance substance can suitably be in the form of a liquid, gel or solid. The fragrance substance is preferably in the form of a liquid. Suitable solvents for the fragrance substances include those known in the art such as water, propanol, ethanol and appropriate ethers, carbitols and glycols.

Preferably, the fragrance delivery system according to the present invention is adapted to be connected to an electric power supply, for instance, a domestic main socket, to allow the control unit and switching element to function.

These and other aspects of the invention will be apparent from and elucidated with reference to the embodiments described hereinafter. Specific embodiments of the invention are set forth in the dependent claims. Further details, aspects and embodiments of the invention will be described, by way of example only, with reference to the attached drawings.
Figure 1 schematically shows a cross-sectional view of an example of an embodiment of a fragrance delivery device according to present invention.
Figure 2 schematically shows a cross-sectional view of the example of FIG. 1 along the line II-II.
Figure 3 schematically shows a block diagram of a first example of an embodiment of a control unit suitable for the example of FIGs. 1 and 2.
Figure 4 schematically shows a block diagram of a second example of an embodiment of a control unit suitable for the example of FIGs. 1 and 2.
Figure 5 shows a timing diagram of outputs in example of FIG. 4.
Figure 6 schematically shows a perspective view of an example of an embodiment of a cartridge suitable for the example of FIGs. 1 and 2.

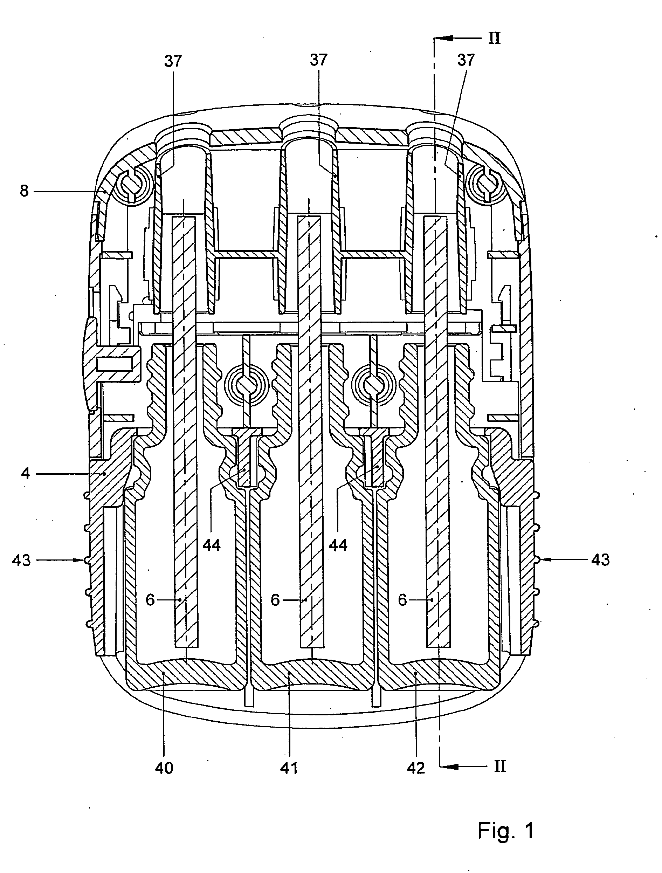

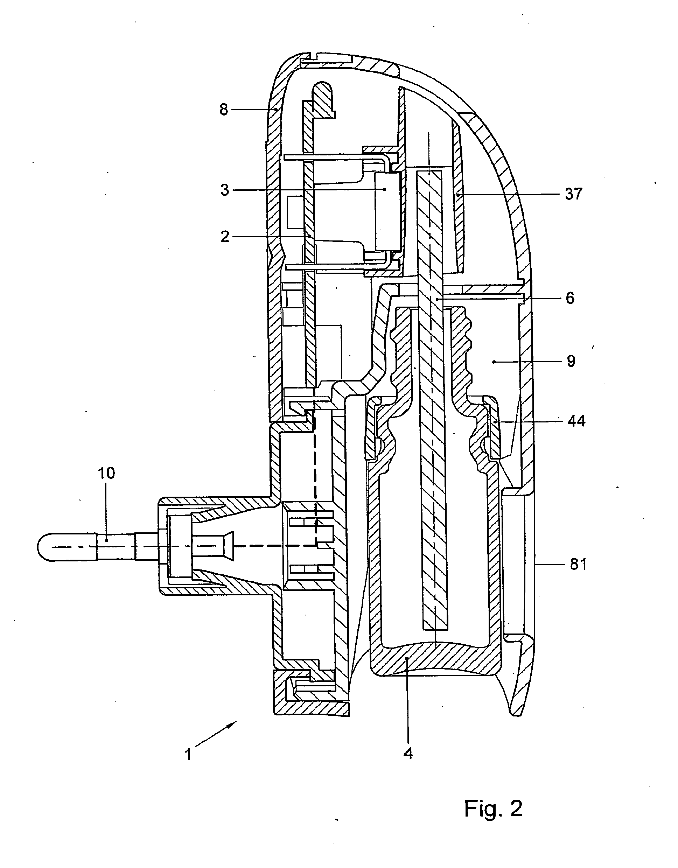

The example of a fragrance delivery system 1 shown in FIGs. 1 and 2 comprises a control unit 2 and a release mechanism 3. The control unit 2 is connected to the release mechanism 3. The release mechanism 3 can, as is explained below in more detail, release one or more selected fragrances. The control unit 2 can control via a suitable control signal which fragrance is selected and released by the release mechanism.

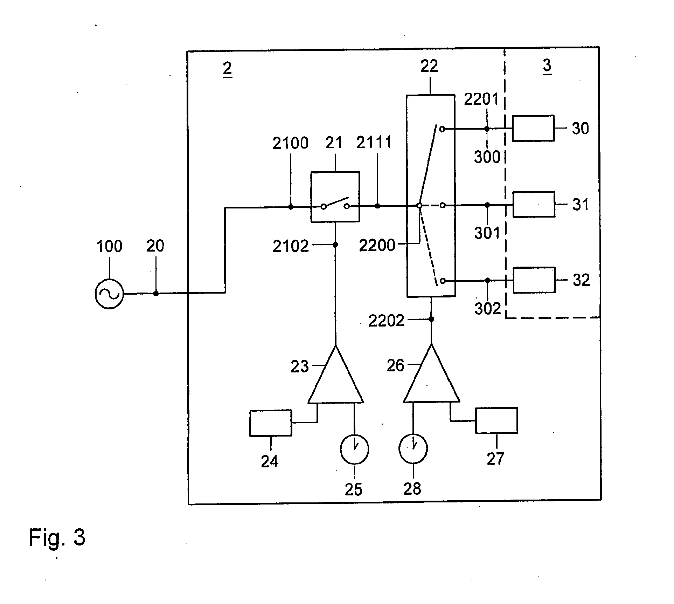

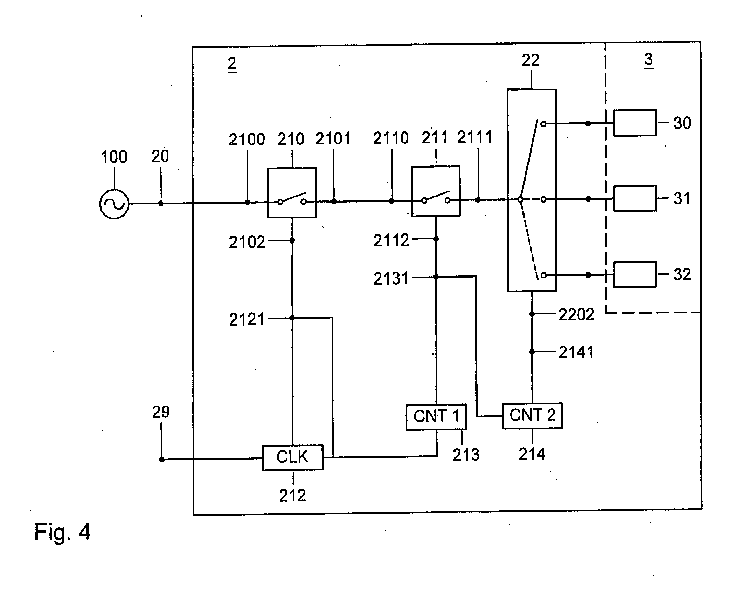

In the example of FIG. 1 and 2 the release mechanism 3 can operate on one or more fragrance reservoirs 40-42 to release one or more selected fragrances from a number of fragrances present in the fragrance delivery system 1. In this example, the release mechanism 3 comprises a number of heating elements 30-32, as is shown more clearly in FIGs. 3 and 4. Each of the heating elements 30-32 is in thermal contact with at least a part of the fragrance chemical in a different one of the fragrance reservoirs 40-42. The heating elements 30-32 may be controlled separately by the control unit 2 to heat the selected fragrance chemical. This heating causes an increased release, e.g. by evaporation or sublimation of the fragrance substance from the respective reservoir 40-42.

The heating element 30-32 may be implemented in any manner suitable for the specific implementation. The heating element may for example be implemented as a resistor or other electro-thermal transducer which can convert electrical energy into thermal energy and supply the thermal energy to the fragrance substance.

The control unit 2 is arranged to select a heating element 30-32 which can heat the selected fragrance. FIG. 3 shows a block diagram of an example of the control unit 2. The example of FIG. 3 comprises a selection unit 22 for selecting the selected fragrance from the fragrances present in the fragrance delivery system 1. E.g. in this example, the selection unit 22 has an input and a plurality of outputs. Each of the outputs is connected to an input 300-302 of another heating element 30-32. The selection unit 22 provides an electrical connection between the input and a selected one of the outputs. Via the selection unit 22 energy, e.g. in this example electrical power, is provided to the selected output and hence to a selected heating element 30-32. Via the heating element the provided energy is used to release the selected fragrance. Accordingly, by controlling the selected output of the selection device 22, the fragrance being released may be selected.

In the examples of FIGs. 3 and 4, the control unit 2 can control the release mechanism 3 to be in a first state or in a second state. In the first state the selected fragrance is released at a higher rate than in the second state. It is found that by varying the rate, the habituation to the fragrance may be reduced. For example, by varying the rate between a first state in which the rate lies above a threshold at which an individual notices said fragrance and a second state in which the rate lies below this threshold, habituation is found be reduced substantially. In the second state, the rate may for example be very low or even zero, thus prolonging the period a reservoir may be used without requiting refills.

In the example of FIG. 3 for instance, the control unit 2 comprises a switching element 21 which in a conducting state allows a current flow between a switch input 2100 and a switch output 2111, whereas in a non-conducting state the current flow between the switch input 2100 and the switch output 2111 is inhibited, or at least less than in the conducting state. Thus, depending on the state of the switch more or less current flows to the selected heating element 30 and accordingly more or less thermal energy is provided to the selected fragrance and accordingly the release rate of the selected fragrance is varied.

The state of the switching element 21 may be controlled by a switch control signal provided at a switch control input 2102. In the example of Fig. 3, the switch control signal is generated by a switch control circuit. The switch control circuit comprises a timer 25 which measures the duration of the state of the switch. The switch control circuit further comprises a memory 24 in which a desired value of the duration of the conducting and/or non-conducting state is stored. A comparator 23 is connected to the memory 24 and the timer 25 and can compare the measured duration with the value stored in the memory 24. When this comparison reveals that the measured period is below the stored value, the comparator outputs a first signal to the switch control input 2102 in response to which the switch is e.g. in the conducting state and when this comparison reveals that the measured period is above the stored value, the comparator outputs a second signal to the switch control input 2102 in response to which the switch is e.g. in the non-conducting state. Thus, the state of the switch and accordingly the state of the selected heating element 30-32 may be controlled.

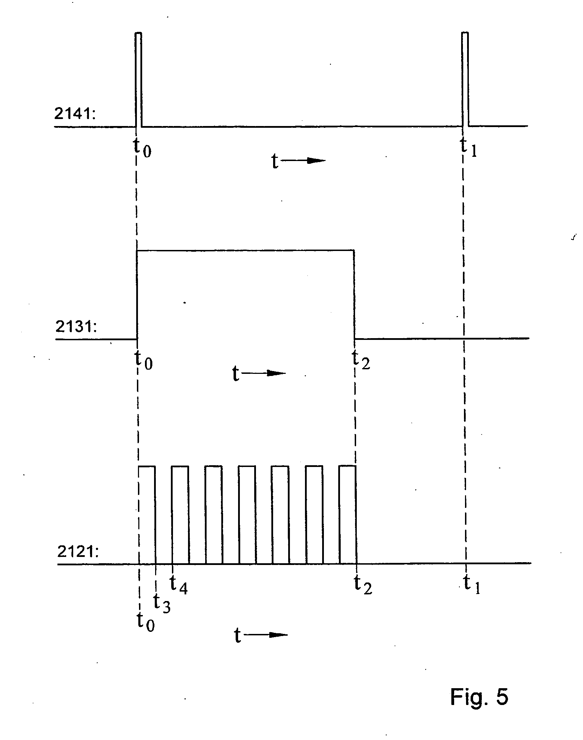

In the example of Fig. 4, the switching element 210, 211 is arranged to switch the release mechanism 3 between the first state and the second state during a first period of time, e.g. between t₀ and t₂ in the timing diagram of FIG. 5. Thereby, the fragrance intensity may be adjusted during the first period to meet more appropriately the size of the space to which the fragrance is to be delivered. For example, by controlling e.g. the duty cycle of the switching between the first state and the second state, the average release rate during the first period may be controlled. Thus, for example, the release rate of the selected fragrance may be adapted to a room in which the fragrance delivery system 1 is provided in a simple manner and the need for complex control elements which for example control the temperature to which the fragrance chemical is heated is obviated. The control of the first state and the second state may be implemented in any manner suitable for the specific implementation. In the example of FIG. 4, for instance, the control circuit has a release control input 29 by means of which the duty cycle of the signal generated by the clock 212 may be controlled, for example manually.

During the first period, the release mechanism 3 may be switched between the first state and the second state in an alternating manner. A cycle of the first state and second state may have any period suitable for the specific implementation. For example, the duration of the first state may be in the range of from 100 to 200 seconds, and the duration of the second state may be in the range of from 20 to 100 seconds. The ratio of the duration of the first state relative to the duration of the second state may have any value suitable for the specific implementation and for example be in the range of 2 to 5 during the first period.

The switching element 21 may further be arranged to switched the release mechanism 3 between the first state and the second state in a different manner during a second period, e.g. between t₂ and t₁. In the example o FIG. 4, during the second period of time for instance, the ratio of the duration of the first state relative to the duration of the second state may lower than in the first period of time. For instance, as shown in FIG. 5 during the second period the first state may be absent, e.g. the ratio is (almost) zero. In FIG. 5, the output signals of the counter 214 (at output 2141), the counter 213 (at output 2131), and the selection unit 22 (at output 2202) are shown respectively as a function of time t.

In the example of Fig. 4, for instance, the switching element 21 comprises a first switch 210 which is alternately switched between a conducting state and a non-conducting state with a switching period T much shorter than the first period. The state of the first switch 210 is controlled by a suitable control signal provided to a control input 2102 of the switch 210 by a clock 212.

The switching element 21 further has a second switch 211 which is alternately switched between a conducting state and a non-conducting state by a control signal provided at a control input 2112. The control signal is presented to the control input 2112 at a counter output 2131 of a counter 213. An input of the counter 213 is connected to the output 2121 of the clock 212. The counter 213 controls the second switch 211 to be in the conducting state during a period of time corresponding to the first period and in the non-conducting state during a period of time corresponding to the second period. The counter 213 may for example count the number of periods of the clock signal and switch the second switch to the non-conducting state or vice versa after a number of periods corresponding to the duration of the first period. In During the second period of time, the release mechanism may for instance be in the second state only. In the example of FIG. 4, the second switch 211 inhibits a current to the release mechanism 3 during the entire second period and hence controls the release mechanism to be in the second state with a release rate of substantially zero.

Preferably, the second period is sufficiently long to reduce the detection level of a human being, such as to the level before habituation, whereas the first period may be sufficiently short to prevent rising the detection level above the concentration of the selected fragrance in the space in which the fragrance delivery system 1 is positioned. Suitable values for the combined first and second period are found to be in the range of 15 to 60 minutes. The first period has suitably a longer duration than said second period. For example, the first period may have a duration of more than 15 minutes, such as between 20 and 60 minutes, and/or in less than or equal to 60 minutes. The second period may for example have a duration of less than 30 minutes, such as between 10 and 25 minutes.

The first period and said second period may for example follow in an alternating manner. For example, a number of cycles of alternating the first period and the second period may be performed. The duration of the first period and/or second period may differ per cycle or be constant in time.

In the example of FIG. 3 and 4, the selection unit is arranged to periodically select a selected fragrance. In the example of FIG. 3, to that end the selection unit has a control input 2202 which is connected to an output of a comparator 26. The comparator 26 has an input which is connected to a memory 27 and another input is connected to a timer 28. In the memory 27 a desired value of a period of time during which a fragrance is selected may be stored and by means of the comparator 26 a duration measured by the timer 28 may be determined. In case the measured duration exceeds the desired value, the selection unit 22 is controlled by the comparator 26 to select another fragrance. In this respect, the comparator may select a fragrance which is not presently selected or select the presently selected fragrance again.

The selection unit 22 may be arranged to select the fragrances in a predetermined order. In such case, for example, the fragrances may be selected to generate a certain atmosphere or user experience. However, it is also possible to select the fragrances at random, thereby further reducing the habituation of an individual to the fragrances.

The selection unit 22 may for instance be arranged to select a selected fragrance when a second period expires. In the example of Fig. 4, for example, a control input 2202 of the selecting unit 22 is connected to an output 2141 of a counter 214. The counter 214 counts the periods of the signal outputted by the counter 213 connected the second switch 211. In case the output signal of the counter 213 has completed one cycle of a first period and a second period, the counter outputs, as shown in Fig. 5, a control signal to the selection unit 22 in response to which the selection unit 22 selects a heating element 30-32 to provide a current and hence selects a fragrance.

The selection unit may be arranged to select randomly the selected fragrance from the at least two fragrances in case the delivery system 1 is turned on. Thereby the different fragrances are used more and the change that one reservoir is empty before the other reservoirs is reduced.

Returning to Figs. 1 and 2, the control unit 2 is provided in a housing 8. The control unit 2 may be connected by means of a plug 10 to a conventional (not shown) socket and hence to a power source 100.

In the example of Figs. 1 and 2, the release mechanism 3 further comprises a wick 6 which can transport the fragrance out of the reservoir 40-42 into the vicinity of a respective heating element 30-32. The wick 6 may be made of natural or synthetic fibrous materials such as fibreglass, cotton, graphite, mineral fibres, and polyester. The wick extends into a passage 37, which may also be referred to as a chimney. The heating element 30-32 is provided on the outside of the passage 37 and in thermal contact with the inside thereof. The heating element 30-32 may be of any form, for instance, in the form of a ring or a coil around the wick means. Thermal energy is transported from the heating element to the inside of the passage 37 via the passage wall. The thermal energy in the inside of the passage 37 heats the fragrance transported by the wick 6 and thus volatilizes the fragrance. The volatilized fragrance may subsequently be released out of the fragrance deliver system via an open side at the top of the passage 37

It should be noted that the release mechanism 3 may also be implemented in a different manner and for example comprise a pressurizing means which provides a pressure into a reservoir and thereby pumps the fragrance out of the reservoir via a vent.

The fragrance chemical may be of any type suitable for the specific implementation. In case the release mechanism heats the fragrance substance, the fragrance substance may for instance be of a type with a low release at room temperature and exhibit an increased evaporation or sublimation at elevated temperatures (i.e. above room temperature). Such a fragrance substance may be volatilized by increasing the temperature above room temperature, such as to 60° Celsius of higher, such as to 70° Celsius or higher, such as to 75° Celsius or higher, 80° Celsius or higher for example. The fragrance may be provided in the reservoirs 40-42 in any phase. The reservoirs 40-42 for may for example be contain a fluid which comprises the fragrance substance. The fragrance substance can be in the form of a liquid, gel or solid. The fragrance substance is preferably in the form of a liquid. Suitable solvents for the fragrance substances comprise those known in the art such as water, propanol, ethanol and appropriate ethers, carbitols and glycols.

In the example of Figs. 1 and 2, the fragrance delivery system further comprises a holder 9 for holding the two fragrance reservoirs 40-42. In the example, the holder 9 comprises a recess in the housing 8 in which the fragrance reservoirs 40-42 may be placed and be fixated in position relative to the housing 8. When one or more of the fragrance reservoirs 40-42 are empty or when replacing or removing the fragrance reservoirs is desired for other reasons, the fragrance reservoirs 40-42 may be released from the fixated position, in this example manually by pressing clamp constructions 43 which engage on the fragrance reservoirs to lock the fragrance reservoirs 40-42 in position.

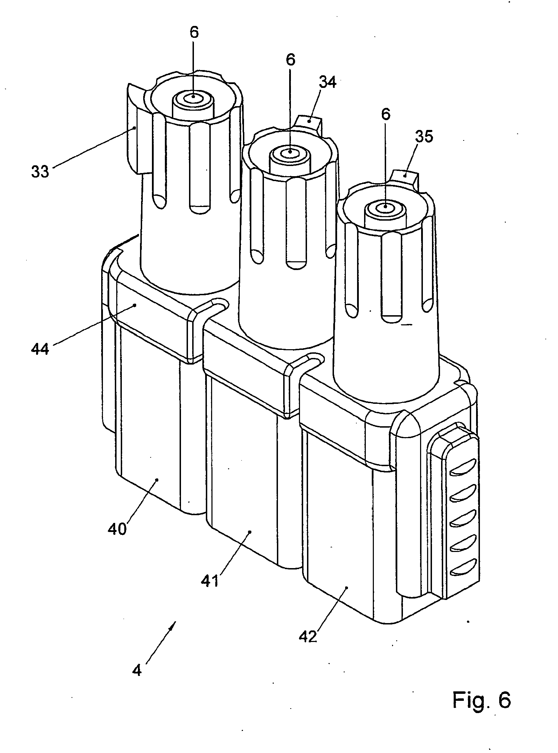

In the example of Figs. 1 and 2, the release mechanism 3 is incorporated in the housing 8. In case one or more reservoirs are removed from the housing, the release mechanism is not removed. Accordingly, the construction of the reservoirs is more simple. However, as shown in FIG. 6, at least a part of the release mechanism may be provided on the reservoirs 40-42. in the example of Fig. 6, heating elements 33-36 in the form of resistors are placed on the outside of the reservoirs 40-42. When the reservoirs 40-42 are placed in the housing 8, the heating elements 33-36 are electrically connected to the control unit 2 and the power source, for example by exposed metal contacts in the housing and on the heating elements.

Preferably, the fragrance delivery system according to the present invention is adapted to be connected to an electric power supply, for instance, a domestic main socket, to allow the control unit and switching element to function.

The at least two fragrances may differ from each other completely or they may be related to each other. It will be understood that each fragrance may be composed of a number of fragrance components. Suitably, the at least two fragrances are related to each other. For instance, a first fragrance may be associated with oriental bamboo, whereas a second fragrance associated with relaxing incense. The fragrances to be used may be selected from a group of types of scents. The fragrances can, for instance, share a common theme. In a very attractive embodiment of the present invention the fragrances complement each other. In the context of the present invention the term "complement" means that the fragrances used convey to a particular theme in the sense that it provides a special physiological effect. The themes can relate to various times of the day, particular geographical regions such as the Orient, seasons of the year, and particular aromas such as vanilla-related fragrances. Suitable themes comprise, for instance, "Oriental Dreams" which an individual will associate with tropical fragrances, "Heavenly Flowers" which an individual will associate with floral fragrances, and "Seaside Stroll" which an individual will associate with fragrances experienced at the seashore.

The present invention also relates to a kit of parts comprising the fragrance delivery system according to the present invention and at least two fragrance reservoirs filled with a fragrance chemical. As shown in the example of FIGs. 1 and 2 and of FIG. 6, the fragrance reservoirs can be part of a fragrance cartridge 4. In these example the cartridge 4 includes a carrier 44 which fixates a number of fragrance reservoirs 40-42 to each other.

The bottles may for example be made of glass or a plastic material. In the example of FIGs. 1 and 2, the fragrance reservoirs are formed as bottles of an optically transparent material. The housing 8 is provided with one or more windows 81 via which the content of the bottles may be viewed.

The present invention further relates to the use of a control unit for controlling a release mechanism to release a selected one of at least two fragrances in a fragrance delivery system, which control unit comprises a selection unit for selecting said selected fragrance and controlling said release mechanism to release said selected fragrance, and a switching element for switching said release mechanism between an active state and an inactive state or vice versa.

In addition, the present invention relates to the use of the fragrance delivery system according to the present invention or the kit of parts according to the present invention to deliver a fragrance to a space which is at least periodically occupied by an individual.

In the foregoing specification, the invention has been described with reference to specific examples of embodiments of the invention. It will, however, be evident that various modifications and changes may be made therein without departing from the broader scope of the invention as set forth in the appended claims. For example, the switches shown in the examples may be implemented as transistors or other suitable electronic components. Also, the fragrance delivery system may be capable of releasing at least three different fragrances in an alternating manner and include a housing which is adapted to hold three different reservoirs.

Furthermore, the control unit may be implemented in non-programmable hardware or as one or more programmable devices or units able to perform the desired device functions by operating in accordance with suitable program code. Furthermore, the devices may be physically distributed over a number of apparatuses, while functionally operating as a single device. For example, the control unit may be implemented as a circuit of discrete electronic components. Also, devices functionally forming separate devices may be integrated in a single physical device. For example, the control unit may be implemented as a single integrated circuit.

## Claims

1. A fragrance delivery system (1), comprising: a control unit (2) for controlling a release mechanism (3) to release at least one selected fragrance, said control unit (2) comprising a selection unit (22) for selecting said selected fragrance from at least two fragrances and controlling said release mechanism to release said selected fragrance, and a switching element (21, 210, 211) for switching said release mechanism (3) between an first state and an second state or vice versa, in which first state said release mechanism (3) releases said selected fragrance at a higher rate than in said second state, **characterised in that** said switching element (21; 210, 211) is arranged to switch said release mechanism (3) between said first state and said second state during a first period of time, and to switch said release mechanism between said first state and said second state during a second period of time, in which second period of time the ratio of the duration of the first state relative to the duration of the second state is lower than in the first period of time, wherein said first period and said second period follow in an alternating manner, and wherein the second period is sufficiently long to reduce the detection level of a human being, such as to the level before habituation.

2. A delivery system according to claim 1, wherein the first period is sufficiently short to prevent rising the detection level above the concentration of the selected fragrance in the space in which the fragrance delivery system is positioned.

3. A delivery system according to any one of claims lor 2, which further comprises a timer (25, 28) for measuring a period of time of said first state and/or second state, a memory element (24, 27) in which at least one desired value of a period of the first and/or second state may be stored, and a comparator (23, 26) for comparing the measured period with said desired value and outputting a switching signal to said switching element (21), and wherein said switching element is arranged to change said state in response to said switching signal.

4. A delivery system according to any one of claims 1, 2 or 3, wherein said
selection unit (22) is arranged to periodically select, in response to a selection signal, instead of a presently selected fragrance another fragrance, which delivery
system (1) further comprises a timer (28) for measuring a period of time during which
said presently selected fragrance has been released, and a comparator (26) for comparing said measured period with a predetermined value representing a desired period and outputting said selection signal to the selection unit

5. A delivery system according to claim 4, wherein the timer (28) is capable of measuring a period of time in the range of from 25 to 45 minutes.

6. A delivery system according to claim 1 or 2, wherein said first period has a longer duration than said second period.

7. A delivery system according to any one of claims 1-3, wherein during the second period of time said release mechanism (3) is in the second state only.

8. A delivery system according to any one claims 1-4, wherein in the second state said release mechanism (3) releases said selected fragrance at a rate below a threshold at which an individual notices said fragrance, such as a rate of substantially zero.

9. A delivery system according to any one of claims 1-8, wherein said selection unit (22) is arranged to periodically select a selected fragrance.

10. A delivery system according to claim 9, wherein said selection unit (22) is arranged to select a selected fragrance when a second period expires.

11. A delivery system according to any one of claims 1-10, wherein said release mechanism (3) comprises an electro-thermal transducer (33-36), said transducer being electrically connectable to an electrical power source (100) and which transducer may be coupled thermally to a fragrance chemical, for converting electrical energy from said power
source to thermal energy applied to said selected fragrance.

12. A delivery system according to any one of claims 1-11, further comprising a holder (44) for holding at least two fragrance reservoirs (40-42).

13. A delivery system according to claim 12 further comprising a release mechanism (3) engageable on said fragrance reservoirs (40-42), for releasing a selected one
of at least two fragrances from said fragrance reservoir.

14. A delivery system according to any one of claims 3-13, wherein the timer (25) is capable of measuring a period of time of said first state in the range of from 100 to 200 seconds.

15. A delivery system according to any one of claims 6-14, the timer (25) is capable of measuring a period of time of said second state the range of from 20 to 100 seconds.

16. A kit of parts comprising the device according to any one of claims 1-15 and at least two fragrance reservoirs (40-42) filled with a fragrance chemical.

17. A kit of parts according to claim 16, wherein the at least two fragrance reservoirs (40-42) are each provided with a release mechanism (3) to release the fragrance contained in the fragrance reservoir.

18. A kit of parts according to claim 16 or 17, wherein the at least two fragrance reservoirs (40-42) are part of a fragrance cartridge (4).

19. A kit of parts according to any one of claims 16-18, wherein the at least two fragrances complement each other.

20. Use of a control unit (2) for controlling a release mechanism (3) to release a selected one of at least two fragrances in a fragrance delivery system (1), which control unit (2) comprises a selection unit (22) for selecting said selected fragrance and controlling said release mechanism (3) to release said selected fragrance, and a switching element (21; 210, 211) for switching said release mechanism (3) between an active state and an inactive state or vice versa, wherein said switching element (21; 210, 211) is arranged to switch said release mechanism (3) between said active state and said inactive state during a first period of time, and to switch said release mechanism between said active state and said inactive state during a second period of time, in which second period of time the ratio of the duration of the active state relative to the duration of the inactive state is lower than in the first period of time, wherein said first period and said second period follow in an alternating manner, and whereby the second period is sufficiently long to reduce the detection level of a human being, such as to the level before habituation.

21. Use of a fragrance delivery system according to any one of claims 1-15 to deliver a fragrance to a space which is at least periodically occupied by an individual.

## Patentansprüche

1. Duftstoffabgabesystem (1), umfassend: eine Steuereinheit (2) zum Steuern eines Freisetzungsmechanismus (3) zur Freisetzung mindestens eines ausgewählten Duftstoffs, wobei die Steuereinheit (2) eine Auswahleinheit (22) zum Auswählen des ausgewählten Duftstoffs aus mindestens zwei Duftstoffen und zum Steuern des Freisetzungsmechanismus zur Freisetzung des ausgewählten Duftstoffs umfasst und ein Schaltelement (21; 210, 211) zum Schalten des Freisetzungsmechanismus (3) zwischen einem ersten Zustand und einem zweiten Zustand oder umgekehrt, wobei in dem ersten Zustand der Freisetzungsmechanismus (3) den ausgewählten Duftstoff bei einer höheren Rate freisetzt als in dem zweiten Zustand, **dadurch gekennzeichnet, dass** das Schaltelement (21; 210, 211) so eingerichtet ist, dass es den Freisetzungsmechanismus (3) während eines ersten Zeitabschnitts zwischen dem ersten Zustand und dem zweiten Zustand schaltet und den Freisetzungsmechanismus während eines zweiten Zeitabschnitts zwischen dem ersten Zustand und dem zweiten Zustand schaltet, wobei in dem zweiten Zeitabschnitt das Verhältnis der Dauer des ersten Zustands relativ zu der Dauer des zweiten Zustands geringer ist als in dem ersten Zeitabschnitt, wobei der erste Zeitabschnitt und der zweite Zeitabschnitt abwechselnd aufeinander folgen und wobei der zweite Zeitabschnitt lang genug ist, um das Wahrnehmungsniveau eines Menschen zu verringern, wie etwa auf das Niveau vor einer Gewöhnung.

2. Abgabesystem nach Anspruch 1, wobei der erste Zeitabschnitt kurz genug ist, um zu verhindern, dass das Wahrnehmungsniveau über die Konzentration des ausgewählten Duftstoffs in dem Raum, in dem das Duftstoffabgabesystem angeordnet ist, ansteigt.

3. Abgabesystem nach einem der Ansprüche 1 oder 2, welches ferner einen Zeitmesser (25, 28) zum Messen eines Zeitabschnitts des ersten Zustands und/oder des zweiten Zustands, ein Speicherelement (24, 27), in dem mindestens ein gewünschter Wert eines Zeitabschnitts des ersten und/oder zweiten Zustands gespeichert werden kann, und einen Komparator (23, 26) zum Vergleichen des gemessenen Zeitabschnitts mit dem gewünschten Wert und Ausgeben eines Schaltsignals an das Schaltelement (21) umfasst, und wobei das Schaltelement so eingerichtet ist, dass es den Zustand als Reaktion auf das Schaltsignal ändert.

4. Abgabesystem nach einem der Ansprüche 1, 2 oder 3, wobei die Auswahleinheit (22) so eingerichtet ist, dass sie periodisch als Reaktion auf ein Auswahlsignal statt eines gegenwärtig ausgewählten Duftstoffs einen anderen Duftstoff auswählt, wobei
das Abgabesystem (1) ferner einen Zeitmesser (28) zum Messen eines Zeitabschnitts umfasst, während dessen
der gegenwärtig ausgewählte Duftstoff freigesetzt wurde, und einen Komparator (26) zum Vergleichen des gemessenen Zeitabschnitts mit einem vorbestimmten Wert, der einen gewünschten Zeitabschnitt darstellt, und zum Ausgeben des Auswahlsignals an die Auswahleinheit.

5. Abgabesystem nach Anspruch 4, wobei der Zeitmesser (28) in der Lage ist, einen Zeitabschnitt im Bereich von 25 bis 45 Minuten zu messen.

6. Abgabesystem nach Anspruch 1 oder 2, wobei der erste Zeitabschnitt länger dauert als der zweite Zeitabschnitt.

7. Abgabesystem nach einem der Ansprüche 1-3, wobei sich der Freisetzungsmechanismus (3) während des zweiten Zeitabschnitts nur in dem zweiten Zustand befindet.

8. Abgabesystem nach einem der Ansprüche 1-4, wobei der Freisetzungsmechanismus (3) in dem zweiten Zustand den ausgewählten Duftstoff bei einer Rate unterhalb einer Schwelle freisetzt, bei der eine Person den Duftstoff wahrnimmt, wie einer Rate von im Wesentlichen null.

9. Abgabesystem nach einem der Ansprüche 1-8, wobei die Auswahleinheit (22) so eingerichtet ist, dass sie periodisch einen ausgewählten Duftstoff auswählt.

10. Abgabesystem nach Anspruch 9, wobei die Auswahleinheit (22) so eingerichtet ist, dass sie einen ausgewählten Duftstoff auswählt, wenn ein zweiter Zeitabschnitt abläuft.

11. Abgabesystem nach einem der Ansprüche 1-10, wobei der Freisetzungsmechanismus (3) einen elektrothermischen Wandler (33-36) umfasst, wobei der Wandler mit einer elektrischen Stromquelle (100) elektrisch verbunden werden kann und wobei der Wandler mit einer Duftstoffchemikalie thermisch gekoppelt werden kann, um elektrische Energie aus der Stromquelle in Wärmeenergie umzuwandeln, die auf den ausgewählten Duftstoff angewendet wird.

12. Abgabesystem nach einem der Ansprüche 1-11, ferner umfassend einen Halter (44) zum Halten von mindestens zwei Duftstoffbehältern (40-42).

13. Abgabesystem nach Anspruch 12, ferner umfassend einen Freisetzungsmechanismus (3), der an den Duftstoffbehältern (40-42) einrastbar ist, zur Freisetzung eines ausgewählten von mindestens zwei Duftstoffen aus dem Duftstoffbehälter.

14. Abgabesystem nach einem der Ansprüche 3-13, wobei der Zeitmesser (25) in der Lage ist, einen Zeitabschnitt des ersten Zustands im Bereich von 100 bis 200 Sekunden zu messen.

15. Abgabesystem nach einem der Ansprüche 6-14, wobei der Zeitmesser (25) in der Lage ist, einen Zeitabschnitt des zweiten Zustands im Bereich von 20 bis 100 Sekunden zu messen.

16. Teilesatz, umfassend die Vorrichtung nach einem der Ansprüche 1-15 und mindestens zwei Duftstoffbehälter (40-42), die mit einer Duftstoffchemikalie gefüllt sind.

17. Teilesatz nach Anspruch 16, wobei die mindestens zwei Duftstoffbehälter (40-42) jeweils mit einem Freisetzungsmechanismus (3) versehen sind, um den in dem Duftstoffbehälter enthaltenen Duftstoff freizusetzen.

18. Teilesatz nach Anspruch 16 oder 17, wobei die mindestens zwei Duftstoffbehälter (40-42) Bestandteil einer Duftstoffkartusche (4) sind.

19. Teilesatz nach einem der Ansprüche 16-18, wobei die mindestens zwei Duftstoffe einander ergänzen.

20. Verwendung einer Steuereinheit (2) zum Steuern eines Freisetzungsmechanismus (3) zur Freisetzung eines ausgewählten von mindestens zwei Duftstoffen in einem Duftstoffabgabesystem (1), wobei die Steuereinheit (2) eine Auswahleinheit (22) zum Auswählen des ausgewählten Duftstoffs und zum Steuern des Freisetzungsmechanismus (3) zur Freisetzung des ausgewählten Duftstoffs umfasst und ein Schaltelement (21; 210, 211) zum Schalten des Freisetzungsmechanismus (3) zwischen einem aktiven Zustand und einem inaktiven Zustand oder umgekehrt, wobei das Schaltelement (21; 210, 211) so eingerichtet ist, dass es den Freisetzungsmechanismus (3) während eines ersten Zeitabschnitts zwischen dem aktiven Zustand und dem inaktiven Zustand schaltet und den Freisetzungsmechanismus während eines zweiten Zeitabschnitts zwischen dem aktiven Zustand und dem inaktiven Zustand schaltet, wobei in dem zweiten Zeitabschnitt das Verhältnis der Dauer des aktiven Zustands relativ zu der Dauer des inaktiven Zustands geringer ist als in dem ersten Zeitabschnitt, wobei der erste Zeitabschnitt und der zweite Zeitabschnitt abwechselnd aufeinander folgen und wobei der zweite Zeitabschnitt lang genug ist, um das Wahrnehmungsniveau eines Menschen zu verringern, wie etwa auf das Niveau vor einer Gewöhnung.

21. Verwendung eines Duftstoffabgabesystems nach einem der Ansprüche 1-15 zum Abgeben eines Duftstoffs in einen Raum, in dem sich wenigstens periodisch eine Person aufhält.

## Revendications

1. Système de libération de parfum (1), comprenant : une unité de contrôle (2) pour contrôler un mécanisme de libération (3) permettant de libérer au moins un parfum choisi, ladite unité de contrôle (2) comprenant une unité de sélection (22) permettant de sélectionner ledit parfum choisi parmi au moins deux parfums et contrôler ledit mécanisme de libération pour libérer ledit parfum choisi, et un élément de commutation (21 ; 210, 211) destiné à commuter ledit mécanisme de libération (3) entre un premier état et un deuxième état ou vice-versa, premier état dans lequel ledit mécanisme de libération (3) libère ledit parfum choisi à un taux plus élevé que dans ledit deuxième état, **caractérisé en ce que** ledit élément de commutation (21 ; 210, 211) est arrangé pour commuter ledit mécanisme de libération (3) entre ledit premier état et ledit deuxième état durant une première période de temps, et pour commuter ledit mécanisme de libération entre ledit premier état et ledit deuxième état durant une deuxième période de temps, deuxième période de temps dans laquelle le rapport de la durée du premier état par rapport à la durée du deuxième état est plus bas que dans la première période de temps, ladite première période et ladite deuxième période se suivant de manière alternée, et la deuxième période étant suffisamment longue pour réduire le niveau de détection d'un être humain, tel que jusqu'au niveau avant accoutumance.

2. Système de libération selon la revendication 1, dans lequel la première période est suffisamment courte pour empêcher une augmentation du niveau de détection au-dessus de la concentration du parfum choisi dans l'espace dans lequel le système de libération de parfum est positionné.

3. Système de libération selon l'une quelconque des revendications 1 ou 2, qui comprend en outre une minuterie (25, 28) pour mesurer une période de temps dudit premier état et/ou deuxième état, un élément de mémoire (24, 27) dans lequel au moins une valeur souhaitée d'une période du premier et/ou deuxième état peut être stockée, et un comparateur (23, 26) pour comparer la période mesurée à ladite valeur souhaitée et produire un signal de commutation audit élément de commutation (21), et dans lequel ledit élément de commutation est arrangé pour changer ledit état en réponse audit signal de commutation.

4. Système de libération selon l'une quelconque des revendications 1, 2 ou 3, dans lequel ladite
unité de sélection (22) est arrangée pour sélectionner périodiquement, en réponse à un signal de sélection, au lieu d'un parfum actuellement choisi un autre parfum, lequel système de libération
(1) comprend en outre une minuterie (28) pour mesurer une période de temps durant laquelle
ledit parfum actuellement choisi a été libéré, et un comparateur (26) pour comparer ladite période mesurée à une valeur prédéterminée représentant une période souhaitée et envoyer ledit signal de sélection à l'unité de sélection.

5. Système de libération selon la revendication 4, dans lequel la minuterie (28) est susceptible de mesurer une période de temps dans l'intervalle allant de 25 à 45 minutes.

6. Système de libération selon la revendication 1 ou 2, dans lequel ladite première période a une durée plus longue que ladite deuxième période.

7. Système de libération selon l'une quelconque des revendications 1 à 3, dans lequel durant la deuxième période de temps, ledit mécanisme de libération (3) est dans le deuxième état uniquement.

8. Système de libération selon l'une quelconque des revendications 1 à 4, dans lequel dans le deuxième état, ledit mécanisme de libération (3) libère ledit parfum choisi à un taux inférieur à un seuil auquel une personne remarque ledit parfum, tel qu'un taux essentiellement nul.

9. Système de libération selon l'une quelconque des revendications 1 à 8, dans lequel ladite unité de sélection (22) est arrangée pour sélectionner périodiquement un parfum choisi.

10. Système de libération selon la revendication 9, dans lequel ladite unité de sélection (22) est arrangée pour sélectionner un parfum choisi lorsqu'une deuxième période expire.

11. Système de libération selon l'une quelconque des revendications 1 à 10, dans lequel ledit mécanisme de libération (3) comprend un transducteur électro-thermique (33-36), ledit transducteur pouvant être connecté électriquement à une source d'alimentation électrique (100) et lequel transducteur peut être couplé thermiquement à une substance chimique de parfum, pour convertir l'énergie électrique de ladite source d'alimentation en énergie thermique appliquée audit parfum choisi.

12. Système de libération selon l'une quelconque des revendications 1 à 11, comprenant en outre un support (44) pour contenir au moins deux réservoirs de parfum (40-42).

13. Système de libération selon la revendication 12, comprenant en outre un mécanisme de libération (3) pouvant venir en prise sur lesdits réservoirs de parfum (40-42), pour libérer un parfum choisi
parmi au moins deux parfums à partir dudit réservoir de parfums.

14. Système de libération selon l'une quelconque des revendications 3 à 13, dans lequel la minuterie (25) est susceptible de mesurer une période de temps dudit premier état dans l'intervalle allant de 100 à 200 secondes.

15. Système de libération selon l'une quelconque des revendications 6 à 14, dans lequel la minuterie (25) est susceptible de mesurer une période de temps dudit deuxième état dans l'intervalle allant de 20 à 100 secondes.

16. Trousse de pièces comprenant le dispositif selon l'une quelconque des revendications 1 à 15 et au moins deux réservoirs de parfum (40-42) remplis avec une substance chimique parfumée.

17. Trousse de pièces selon la revendication 16, dans laquelle les au moins deux réservoirs de parfum (40-42) sont chacun pourvus d'un mécanisme de libération (3) permettant de libérer le parfum contenu dans le réservoir de parfum.

18. Trousse de pièces selon la revendication 16 ou 17, dans laquelle les au moins deux réservoirs de parfum (40-42) font partie d'une cartouche de parfum (4).

19. Trousse de pièces selon l'une quelconque des revendications 16 à 18, dans laquelle les au moins deux parfums se complètent l'un l'autre.

20. Utilisation d'une unité de contrôle (2) pour contrôler un mécanisme de libération (3) permettant de libérer un parfum choisi parmi au moins deux parfums dans un système de libération de parfum (1), laquelle unité de contrôle (2) comprend une unité de sélection (22) pour sélectionner ledit parfum choisi et contrôler ledit mécanisme de libération (3) pour libérer ledit parfum choisi, et un élément de commutation (21 ; 210, 211) destiné à commuter ledit mécanisme de libération (3) entre un état actif et un état inactif ou vice-versa, dans laquelle ledit élément de commutation (21 ; 210, 211) est arrangé pour commuter ledit mécanisme de libération (3) entre ledit état actif et ledit état inactif durant une première période de temps, et pour commuter ledit mécanisme de libération entre ledit état actif et ledit état inactif durant une deuxième période de temps, deuxième période de temps dans laquelle le rapport de la durée de l'état actif par rapport à la durée de l'état inactif est plus bas que dans la première période de temps, ladite première période et ladite deuxième période se suivant de manière alternée, et la deuxième période étant suffisamment longue pour réduire le niveau de détection d'un être humain, tel que jusqu'au niveau avant accoutumance.

21. Utilisation d'un système de libération de parfum selon l'une quelconque des revendications 1 à 15 pour libérer un parfum à un espace qui est au moins périodiquement occupé par une personne.
